# EUROPÄISCHE PATENTSCHRIFT

(11) **EP 0 584 711 B1**
(45) Veröffentlichungstag und Bekanntmachung des Hinweises auf die Patenterteilung: **04.02.1998**
(21) Anmeldenummer: 93113209.6
(22) Anmeldetag: 18.08.1993
(51) Int. Cl.: C07C 233/20, C23F 11/14, C10M 133/16, C10M 173/00, C10M 173/02

(54) **Alkenylbernsteinsäurederivate als Metallbearbeitungshilfsmittel**
Alkenyl succinic acid derivatives as metal working agents
Dérivés d'acide alkényl succinique comme agent auxiliaire du travail des métaux

(30) Priorität: 22.08.1992 DE 4227938
(43) Veröffentlichungstag der Anmeldung: 02.03.1994
(73) Patentinhaber: Clariant GmbH, 65929 Frankfurt am Main (DE)
(72) Erfinder: Wehle, Detlef, Dr., D-65527 Niedernhausen (DE); Lorke, Horst, D-65835 Liederbach (DE)

(56) Entgegenhaltungen:
- EP-A- 0 074 199
- EP-A- 0 127 132
- EP-A- 0 144 738
- EP-A- 0 328 498
- EP-A- 0 464 473
- DE-A- 3 300 874
- US-A- 4 348 210
- PATENT ABSTRACTS OF JAPAN, vol. 6, no. 102, (C-107)(980), 11. Juni 1982, & JP-A-57 032 383

## Beschreibung

Die Anwendung von Metallbearbeitungshilfsmitteln erstreckt sich auf zahlreiche Bearbeitungsvorgänge der Vor-und Nachbehandlung von Metallen. Das Arbeitsgebiet umfaßt die spangebende und spanlose Verformung von Metallteilen. Bei spangebender Verformung der Metalle werden Bohr- und Schneidöle, bei spanloser Verformung Walz- und Ziehöle eingesetzt.

Korrosionsschutzmittel und Emulgatoren in emulgierbaren Metallbearbeitungsflüssigkeiten sollen folgende Forderungen erfüllen:
1. ausgeprägte Korrosionsschutzeigenschaften gegenüber Eisenmetallen
2. Emulgiervermögen gegenüber Mineralölen, eventuell auch in Kombination mit ausgewählten nichtionogenen Verbindungen
3. geringe Schaumneigung bzw. schneller Schaumzerfall
4. Verhinderung des Wachstums von Mikroorganismen

Diese Forderungen werden von den bereits bekannten Verbindungen nur teilweise erfüllt.

Aus EP-A-127 132 sind Salze von C₆-C₁₂-Alkenylbernsteinsäurehalbamiden und deren Verwendung als Korrosionsschutzmittel bekannt. Diese Verbindungen zeigen jedoch kein ausreichendes Emulgiervermögen, was zu ungenügenden Standzeiten der Gebrauchsemulsion führt. Außerdem neigen Emulsionen mit diesen Verbindungen zur Schaumbildung, was besonders bei Arbeitsprozessen, wie dem Schleifen, von Nachteil ist, wenn die Emulsionen hohen mechanischen Beeinflussungen ausgesetzt sind.

In der DE-A-33 00 874 werden Alkanolaminsalze von C_{S}-C₁₈-Alkenylbernsteinsäurehalbamiden beschrieben, bei denen zur Halbamidbildung ein primäres Amin mit einer C₁-C₁₀-Alkylkette eingesetzt wird.

EP-A-0 074 199 beschreibt Reaktionsprodukte aus einem Bernsteinsäure-Acylierungsmittel und einem Etheramin als Korrosionsschutzmittel in Schmiermitteln und Kraftstoffen.

Die DE-A-33 41 013 beschreibt C₆-C₁₈-Alkenylbernsteinsäurehalbamide, bei denen zur Halbamidbildung sekundäre Amine mit 1 bis 10 Kohlenstoffatomen eingesetzt werden.

Auch durch den Einsatz dieser Verbindungen können die oben angegebenen Forderungen nicht vollständig erfüllt werden.

Aufgabe der Erfindung ist es, Derivate von Alkenylbernsteinsäuren zur Verfügung zu stellen, die die vorstehend beschriebenen Eigenschaften in sich vereinen.

Überraschender Weise wurde nun gefunden, daß diese Aufgabe durch Salze von Alkenylbernsteinsäurehalbamiden, die als Aminkomponente Alkoxyalkylamine enthalten, gegebenenfalls im Gemisch mit Salzen von Alkenylbernsteinsäurehalbestern, die als Estergruppe ein Alkoholamin enthalten, und Alkenylbernsteinsäureimiden gelöst werden kann.

Gegenstand der Erfindung sind Salze von Alkenylbernsteinsäure-alkoxyalkylhalbamiden der Formel la und/oder Ib, 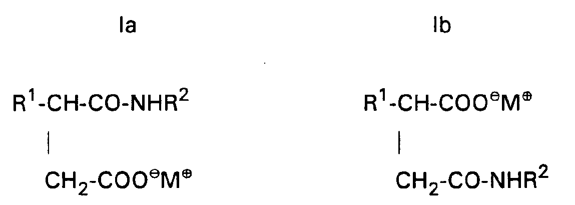 gegebenenfalls im Gemisch mit Salzen von Alkenylbernsteinsäurehalbesteraminen der Formel I la und/oder Ilb 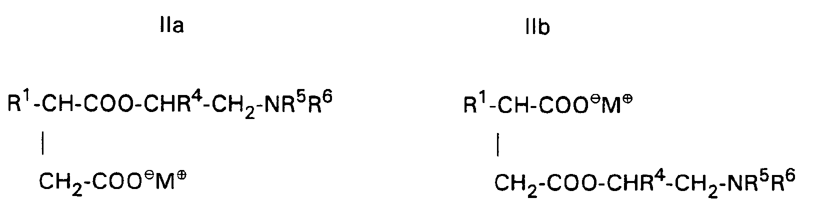 und/oder Alkenylbernsteinsäureimiden der Formel III, 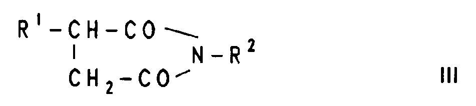 wobei
R¹ = Cₛ-C₃₀-Alkenyl oder C₆-C₃₀-Alkyl, bevorzugt C₉-C₁₈-Alkenyl oder C₉-C₁₈-Alkyl, wobei R¹ geradkettig oder verzweigt sein kann;
R² ₌ -(CH₂)n-O-(A)ₘ-R³ mit
A = -CH₂-CH₂-O- oder -CH(R⁴)-CH₂-O- wobei
R³ = C₁-C₁₈-Alkyl, wobei R³ geradkettig oder verzweigt sein kann,
R⁴ ₌ Wasserstoff oder Methyl,
n = eine Zahl von 1 bis 6,
m = eine Zahl von 0 bis 8 und
M⁺ = ein Alkalimetall- oder Erdalkalimetallkation sowie ein Ammoniumion der Formel HNR⁵R⁶R⁷, wobei R⁵, R⁶ und R⁷ gleich oder verschieden sind und Wasserstoff, C₁-C₆-Alkyl oder Hydroxy-(C₁-C₆)-alkyl, insbesondere 2-Hydroxyethyl und 2-Hydroxypropyl bedeuten.

Die Alkenylbernsteinsäurehalbesteraminsalze der Formel Ila, Ilb und die Alkenylbernsteinsäureimide der Formel III können als Nebenprodukte bei der Herstellung der Alkenylbernsteinsäure-alkoxyalkyl-halbamidsalze der Formel la, Ib auftreten und liegen gewöhnlich in Konzentrationen von 0 bis 40 Gew.-%, bevorzugt 0 bis 20 Gew.- %, im Gemisch mit den Alkenylbernsteinsäurealkoxyalkyl-halbamidsalzen der Formel la, Ib vor.

Ebenfalls Gegenstand der Erfindung ist ein Verfahren zur Herstellung von Salzen von Alkenylbernsteinsäure-alkoxyalkyl-halbamiden der Formel la und/oder Ib, gegebenenfalls in Mischungen mit Salzen von Alkenylbernsteinsäurehalbesteraminen der Formeln Ila und/oder Ilb und/oder Alkenylbernsteinsäureimiden der Formel III, das dadurch gekennzeichnet ist, daß Alkenylbernsteinsäureanhydride mit Alkoxyalkylaminen der Formel IV 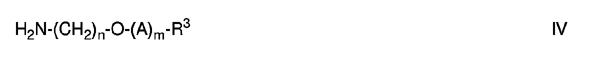 wobei
R³ = C₁-C₁₈-Alkyl, wobei R³ geradkettig oder verzweigt sein kann,
n = eine Zahl von 1 bis 6 und
m = eine Zahl von 0 bis 8,

in einem molaren Verhältnis von 1 : 0,7 bis 2,5, vorzugsweise 1 : 0,8 bis 1,2, bei Temperaturen von 20 bis 50°C umgesetzt werden und diese Umsetzung in Gegenwart von 0,58 bis 0,60 Mol, bezogen auf Alkoxyalkylamin der Formel IV, von Basen erfolgt oder im Anschluß an die Umsetzung 0,58 bis 0,60 Mol, bezogen auf Alkoxyalkylamin der Formel IV, von Basen zugegeben wird.

Als Basen werden Alkalihydroxyde, insbesondere NaOH, Erdalkalihydroxyde sowie Alkanolamine der Formel NR⁵R⁶R⁷ [oder Mischungen daraus] eingesetzt. Üblicherweise werden die Basen in einer Menge von 0,58 bis 0,60 Mol, bezogen auf Alkanolamin, eingesetzt.

Die Herstellung der als Ausgangsmaterialien verwendeten Alkenylbernsteinsäureanhydride erfolgt durch Umsetzung von Olefinen und Maleinsäureanhydrid und ist bekannt. Bevorzugte Olefine sind Oligomere des Ethylens, Propylens und Butylens sowie Olefine mit innenständiger Doppelbindung. Beispielsweise kann n-Dodecenylbernsteinsäureanhydrid durch Umsetzung von n-Dodecen-1 mit Maleinsäureanhydrid bei Temperaturen von 150 bis 230 °C bei einem Druck von 3 bis 10 bar in Gegenwart geeigneter Radikalinhibitoren hergestellt werden. Die so erhaltenen Alkenylbernsteinsäureanhydride können entweder nach Reinigung durch Destillation oder Umkristallisation oder aber direkt zur Herstellung der erfindungsgemäßen Verbindungen eingesetzt werden.

Die Alkoxyalkylamine, z.B. Methoxypropylamin lassen sich durch Anlagerung von Acrylnitril an Alkohole sowie anschließender Hydrierung herstellen.

Die erfindungsgemäßen Salze von Alkenylbernsteinsäure-alkoxyalkyl-halbamiden der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III finden Verwendung als Metallbearbeitungshilfsmittel, insbesondere als Korrosionschutzmittel und Emulgator in wäßrigen und/oder ölhaltigen Formulierungen bzw. Metallbearbeitungsflüssigkeiten.

Ebenfalls Gegenstand der Erfindung sind wasser- und/oder ölhaltige Formulierungen zur Bearbeitung von Metallen, insbesondere zur Verwendung als Kühlschmiermittel, die dadurch gekennzeichnet sind, daß sie die vorstehend genannten Salze von Alkenylbernsteinsäure-alkoxyalkyl-halbamiden der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III enthalten.

Die nach den vorstehend beschriebenen Verfahren hergestellten Alkenylbernsteinsäure-alkoxyalkyl-halbamidsalze der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III, bilden in Wasser klare Lösung und ergeben mit Mineralöl in Wasser leicht emulgierbare Formulierungen. Diese Verbindungen werden in konzentrierter Form als Emulgatoren und Korrosionsschutzmittel in ölhaltigen und/oder in wässrigen Formulierungen eingesetzt. Die Konzentration der Alkenylbernsteinsäure-alkoxyalkylhalbamidsalze der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III, in der Formulierung ist höher als bei der Anwendung und beträgt gewöhnlich 10 bis 80 Gew.-%. Zur spangebenden oder spanlosen Verformung wie z. B. in Bohr-, Schneid-, Zieh- und Walzflüssigkeiten wird die Formulierung mit Wasser verdünnt. Zur Bereitung der Formulierungen werden die erfindungsgemäßen Produkte entweder in die erforderliche Menge Wasser eingerührt oder mit Mineralöl bzw. Mineralöl/Wasser gemischt. Die erhaltenen wäßrigen bzw. ölhaltigen Formulierungen werden üblicherweise vor Anwendung mit Wasser verdünnt bzw. emulgiert. Das Verdünnungsverhältnis beträgt im allgemeinen 1 : 10 bis 1 : 100. Die Anwendungskonzentration der Alkenylbernsteinsäure-alkoxyalkyl-halbamidsalze in Metallbearbeitungsflüssigkeiten, z.B. Bohr-, Schneid-, Zieh- und Walzflüssigkeiten, beträgt im allgemeinen etwa 0,1 bis 10 Gew.-%, vorzugsweise 2 bis 10 Gew.-%. Die genannte Konzentration bezieht sich sowohl auf die Anwendung der Produkte in Wasser als auch in Mineralöl/Wasseremulsionen bei der Metallbearbeitung. Unter Metallbearbeitungsflüssigkeiten sind auch Kühlschmiermittel zu verstehen.

Die Alkenylbernsteinsäure-alkoxyalkyl-halbamidsalze werden in wäßrigen, mineralölhaltigen Metallbearbeitungsflüssigkeiten als Emulgator mit Korrosionsschutz und in wäßrigen, mineralölfreien Metallbearbeitungsflüssigkeiten als Korrosionsschutzmittel eingesetzt. Sie sind zur Mischung mit üblichen Mineralölen, insbesondere mit naphthenbasischen, paraffinbasischen und gemischtbasischen Mineralölen geeignet. Die Formulierung bzw. Metallbearbeitungsflüssigkeiten können zusätzliche Hilfsstoffe zur Optimierung des Emulgierverhaltens und des Korrosionsschutzes enthalten. Dafür werden besonders vorteilhaft Oxethylate der allgemeinen Formel V 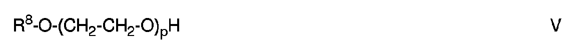 verwendet, wobei R⁸ C₁₀-C₂₂ Alkyl, C₁₀-C₂₂ Alkenyl oder Alkylphenyl mit insgesamt 10 bis 20 C-Atomen und p Zahlen von 2 bis 10 bedeuten.

Auch können Fettsäurealkanolamide der allgemeinen Formel VI 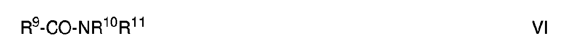 wobei R⁹ C₁₀-C₂₂ Alkyl, C₁₀-C₂₂ Alkenyl und R¹⁰ und R¹¹ gleich oder verschieden sind und Wasserstoff oder Hydroxy-(C₁-C₆)-alkyl, insbesondere 2-Hydroxyethyl oder 2-Hydroxypropyl bedeuten als Hilfsstoffe eingesetzt werden. Weitere geeignete Hilfsstoffe sind Ethercarbonsäuren der Formel VII 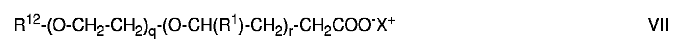 wobei R¹² ein verzweigter oder unverzweigter Cₐ-C₂₂-AlkyI- oder Cₐ-C₂₂-Alkenylrest ist und q und r unabhängig voneinander Werte von 0 bis 10 annehmen können, mit der Maßgabe, daß die Summe aus q und r mindestens 1 ist und X gleich Wasserstoff, ein Alkalimetallatom oder Erdalkalimetallatom, bedeuten.

Ebenfalls geeignete Hilfsstoffe sind alkoxylierte primäre Amine der Formel VIII 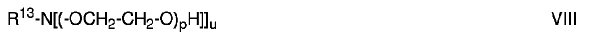 wobei
R¹³ C₁₂-C₂ₒ-Alkyl, oder Alkenyl vorzugsweise C₁₆-C₁₈-Alkyl,
p Zahlen von 2 bis 10, vorzugsweise 4 bis 6 und
u die Zahl 1 oder 2 bedeuten, mit der Maßgabe, daß falls u gleich 1 an der freien Bindung des Stickstoffatoms ein Wasserstoffatom sitzt.

Weitere geeignete Hilfsstoffe sind auch Fettsäurepolyglycolester insbesondere von gesättigten oder ungesättigten Fettsäuren mit 10 bis 22 C-Atomen in der Alkylkette und 1 bis 10 Ethylenoxideinheiten, wie Ölsäure mit 4 bis 6 Ethylenoxideinheiten. Diese Hilfsstoffe sind einzeln oder in Mischungen in den Formulierungen allgemein in Mengen von jeweils ca. 20 bis 40 Gew.-% enthalten. Weiterhin können in den Formulierungen für diese Zwecke übliche Additive wie Entschäumer oder geeignete Konservierungsmittel enthalten sein.

Die nachfolgend zur Charakterisierung verwendeten Begriffe wie Säurezahl, Aminzahl, Basen-Stickstoff und lod-Farbzahl werden wie folgt bestimmt oder ermittelt:

### Bestimmung der Säurezahl:

Zur Bestimmung der Säurezahl, angegeben in mg KOH/g Substanz wiegt man 0,1 bis 0,5 g der Probe in einem 150 ml Becherglas ein, löst in 60 bis 80 ml Ethanol und titriert mit 0,1 N ethanolischer Kaliumhydroxidlösung. Die Endpunktanzeige erfolgt potentiometrisch (Geräte: Metrohm Titroprozessor 682, Dosimat 665 mit kombinierter pH-Glaselektrode).

### Ermittlung des Basen-Stickstoffs und der Aminzahl:

Zur Ermittlung des Basen-Stickstoffs, angegeben in % N, wiegt man 0,1 bis 0,5 g der Probe in einem 150 ml Becherglas ein, löst in 60 bis 80 ml Eisessig und titriert mit 0,1 N Perchlorsäure in Eisessig. Die Endpunktanzeige erfolgt potentiometrisch (Geräte: Metrohm Titroprozessor 682, Dosimat 665 mit kombinierter pH-Glaselektrode). Die Aminzahl, angegeben in mg KOH/g Substanz, berechnet sich aus dem Wert für den Basenstickstoff nach der Formel:
Aminzahl [mg KOH/g] = % Basen-N ^{*} 5,61/0,140067

Die lod-Farbzahl bedeutet die Konzentration an freiem lod in einer lod-lodkaliumlösung (in mg lod je 100 ml lod-lodkaliumlösung) und dient zur Kennzeichnung der Farbe bzw. Lichtdurchlässigkeit dieser Lösung und ähnlichartiger Flüssigkeiten. Grundlage zur Bestimmung der lod-Farbzahl ist die lod-Farbskala (Vergleichsröhrchen). Literatur: DIN 53403.

Die folgenden Ausführungsbeispiele veranschaulichen die vorliegende Erfindung ohne diese zu beschränken:

### Beispiele:

### Beispiel 1

### Herstellung von C_{12/14}-Alkenylbernsteinsäure-3-butoxypropyl-halbamidtriethanolammonium/Na-Salz

In einem 1 L Dreihalskolben mit Rührer, Innenthermometer und Rückflußkühler legte man eine Mischung aus 127,5 g (1,0 Mol) 3-n-Butoxypropylamin, 149,1 g (1,0 Mol) Triethanolamin und 30 g Wasser vor und dosierte innerhalb von 0,5 h 279,2 g (Verseifungszahl: 401,9 mg KOH/g; 1 Mol) techn. C_{12/14}-Alkenylbernsteinsäureanhydrid (Kettenverteilung der Ausgangsolefinmischung: 65 Gew.-% Dodecen, 35 Gew.-% Tetradecen) zu (exotherme Reaktion). Die Innentemperatur wurde durch zeitweiliges Kühlen auf maximal 50°C begrenzt. Nach beendeter Zugabe rührte man noch 2 h bei 50°C nach. Die Reaktionsmischung enthielt zu diesem Zeitpunkt ca. 67 % C_{12/14}-Alkenylbernsteinsäure-3-n-butoxypropylhalbamid-triethanolammonium-/3-butoxypropylammoniumsalz und ca. 33 % C₁₂₁₁₄-Alkenylbernstein- säuretriethanolaminester-3-n-butoxypropylammonium-/triethanolammoniumsalz (ermittelt durch Basen-N-Bestimmung).

Die Reaktionsmischung wurde durch Zusatz von 48 g (0,6 Mol) einer 50 %igen Natronlauge alkalisiert. Man erhielt 642,1 g einer schwach braungelben Flüssigkeit mit lod-Farbzahl 30 bis 40, Aminzahl (potentiometrisch bestimmt) 171 mg KOH/g, Säurezahl 46 mg KOH/g und einer Viskosität von 1,9 Pas (Brookfield, 28°C, 10 U/min).

### Beispiel 2

### Herstellung von C_{12/14}-Alkenylbernsteinsäure-3-methoxypropylhalbamidtriethanolammonium/Na-Salz

In der im Beispiel 1 beschriebenen Apparatur legte man 44,6 g (0,5 Mol) 3-Methoxypropylamin bei 30°C vor und tropfte innerhalb von 30 min 139,6 g (Verseifungszahl: 401,9 mg KOH/g; 0,5 Mol) techn. C_{12/14}-Alkenylbernsteinsäureanhydrid (Kettenverteilung der Ausgangsolefinmischung: 65 Gew.-% Dodecen, 35 Gew.-% Tetradecen) zu (exotherme Reaktion) und hielt die Innentemperatur durch zeitweilige Kühlung bei max. 50°C. Nach beendeter Zugabe rührte man noch 2 h bei 50°C nach (Umsatz laut Kontrolle des Basen-N > 98 %) und neutralisierte durch Zugabe von 74,5 g (0,5 Mol) Triethanolamin. Die Reaktionsmischung wurde abschließend durch Zugabe von 39 g einer 30 %igen Natronlauge alkalisiert (pH-Wert (1 %ig in entmineralisiertem Wasser): 8,4). Man erhielt 296 g einer dunkelgelben Flüssigkeit mit lod-Farbzahl 20, Aminzahl (potentimetrische Bestimmung)
151 mg KOH/g, Säurezahl 50,6 mg KOH/g.

### Beispiel 3

### Herstellung von C_{12/14}-Alkenylbernsteinsäure-3-butoxypropylamidmethyldiethanolammonium/Na-Salz

Zu einer Mischung aus 25,5 g (0,2 Mol) 3-Butoxypropylamin, 23,8 g (0,2 Mol) N-Methyl-diethanolamin und 6 g entmineralisiertem Wasser tropfte man beginnend bei 30°C innerhalb von 30 min 55,8 g (0,2 g) techn. n-C_{12/14}-Alkenylbernsteinsäureanhydrid (exotherme Reaktion), hielt die Innentemperatur durch zeitweilige Kühlung bei 50°C und rührte 2 h bei 50°C nach. Zu diesem Zeitpunkt enthielt die Reaktionsmischung laut Analyse (Basen-N) ca. 86 % n-C₁₂₁₁₄-Alkenylbernsteinsäure-3-butoxypropylhalbamid-triethanolammonium-/3-butoxypropylammoniumsalz und ca. 14 % n-C_{12/14} -Alkenylbernstei nsäure-tri ethanolaminester -3-butoxypropylammonium-ltri ethanolammoniumsalz.

Nach beendeter Reaktion alkalisierte man mit 9,6 g 50 %iger Natronlauge und erhielt so 119,1 g einer gelblichen, viskosen Flüssigkeit mit lod-Farbzahl 20, Aminzahl 168 mg KOH/g und Säurezahl 51 mg KOH/g.

### Beispiel 4

### Herstellung von C₁₂₁₁₄-Alkenylbernsteinsäure-3-methoxypropylamidtriethanolammonium/Na-Salz

Zu 139,8 g (0,5 Mol) n-C_{12/14}-Alkenylbernsteinsäureanhydrid tropfte man innerhalb von 0,5 h unter zeitweiliger Kühlung 44,6 g (0,5 mol) 3-Methoxypropylamin so zu, daß die Innentemperatur 50°C nicht überstieg, ließ 1 h nachrühren, neutralisierte mit einer Mischung aus 75,6 g Triethanolamin und 14,2 g Wasser und erhielt nach 1 h Nachreaktion bei 50°C eine Reaktionsmischung mit einem Halbamid/Ester-Verhältnis von 93:7 (Analyse durch Basen-N). Nach Alkalisierung mit 24 g 50 %iger Natronlauge erhielt man 291,6 g einer dunkelgelben Flüssigkeit mit Aminzahl 160 mg KOH/g, Säurezahl 46 mg KOH/g und einer Viskosität von 2,3 Pas.

### Beispiel 5

### Herstellung von Tripropylbernsteinsäure-3-methoxypropylamidtriethanolammonium/Na-Salz

Zu einer Mischung aus 44,6 g (0,5 Mol) 3-Methoxypropylamin, 74,5 g (0,5 Mol) Triethanolamin und 13,6 g Wasser tropfte man beginnend mt 30°C innerhalb von 0,5 h unter zeitweiliger Kühlung 111,5 g (0,5 Mol) techn. Tripropenylbernsteinsäureanhydrid so zu, daß die Innentemperatur 50°C nicht überstieg und rührte anschließend noch 2 h bei dieser Temperatur nach. Zu diesem Zeitpunkt enthielt die Reaktionsmischung ca. 63 % Halbamid und 37 % Halbester. Nach Alkalisieren mit 24 g 50 %iger Natronlauge erhielt man 266,2 g einer schwach gelben, viskosen Flüssigkeit mit Aminzahl 206 mg KOH/g, Säurezahl 56 mg KOH/g.

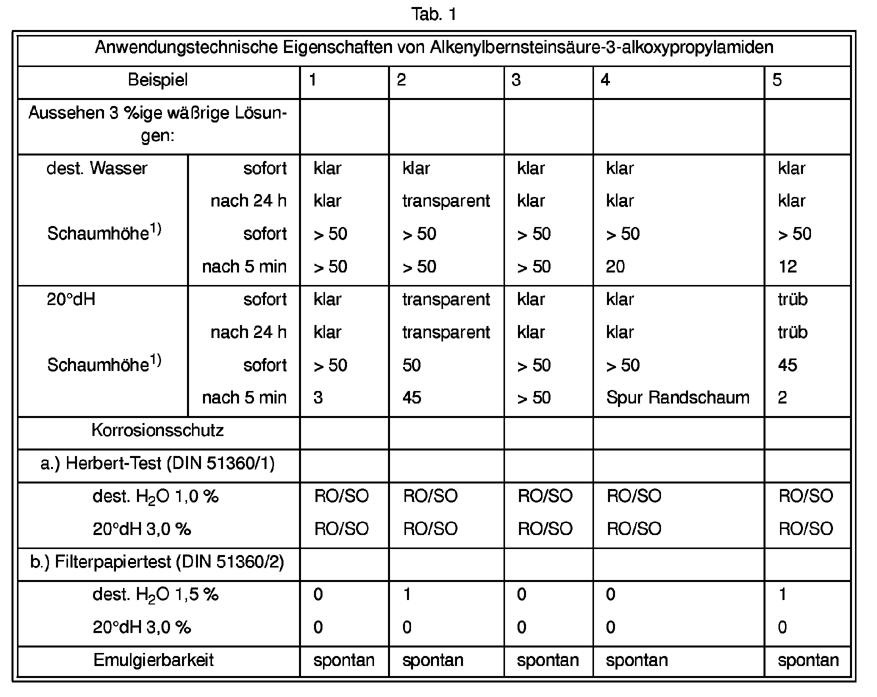

Anmerkungen zu Tabelle 1:
1) Zur Bestimmung des Schaumverhaltens wurden jeweils 50 ml an 3 %igen wäßrigen Lösungen der Versuchsprodukte in einen 100 ml Meßzylinder mit Graduierung gegeben, mit einem Glasstopfen verschlossen und 20 x in horizontaler Richtung kräftig geschüttelt. Die Beurteilung erfolgt nach den angegebenen Wartezeiten durch Messung der Schaummenge in ml.

### Angaben zur Testdurchführung:

Geprüft wurden Formulierungen bestehend aus:
18% Emulgatormischung
4 % Nonylphenolethoxilat
2 % Wasser
78 % naphthenbasisches Mineralöl

Emulgatormischung:
37 % Produkt laut Beispiel
32 % Oleylalkohol + 2EO (Ethylenoxid)
31 % Tallölfettsäurediethanolamid

## Patentansprüche

1. Salze von Alkenylbernsteinsäure-alkoxyalkyl-halbamiden der Formel la und/oder Ib, 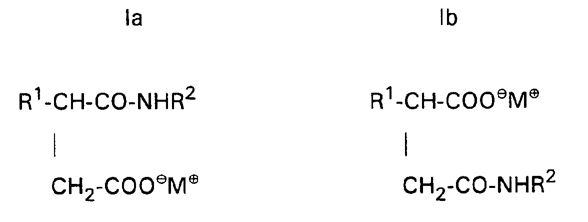 gegebenenfalls im Gemisch mit Salzen von Alkenylbernsteinsäurehalbesteraminen der Formel Ila und/oder Ilb 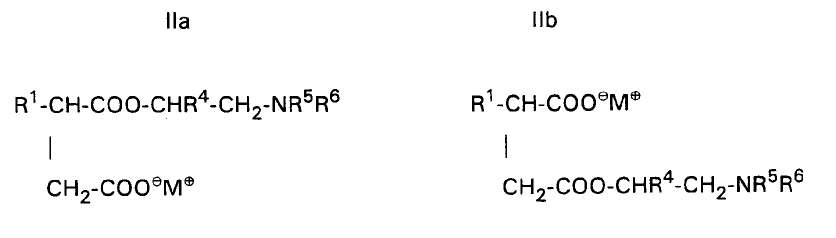 und/oder Alkenylbernsteinsäureimiden der Formel III, 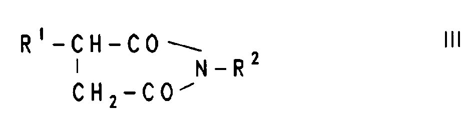 wobei
R¹ = C₆-C₃₀-Alkenyl oder C₆-C₃₀-Alkyl, bevorzugt C₉-C₁₈-Alkenyl oder C₉-C₁₈-Alkyl, wobei R¹ geradkettig oder verzweigt sein kann;
R² = -(CH₂)ₙ-O-(A)ₘ-R³ mit
A = -CH₂-CH₂-O- oder -CH(R⁴)-CH₂-O- wobei
R³ = C₁-C₁₈-Alkyl, wobei R³ geradkettig oder verzweigt sein kann,
R⁴ ₌ Wasserstoff oder Methyl,
n = eine Zahl von 1 bis 6,
m = eine Zahl von 0 bis 8 und
M⁺ = ein Alkalimetall- oder Erdalkalimetallkation sowie ein Ammoniumion der Formel HNR⁵R⁶R⁷, wobei R⁵, R⁶ und R⁷ gleich oder verschieden sind und Wasserstoff, C₁-C₆-Alkyl oder Hydroxy-(C₁-C₆)-Alkyl, insbesondere 2-Hydroxyethyl und 2-Hydroxypropyl bedeuten.

2. Verbindungen gemäß Anspruch 1, dadurch gekennzeichnet, daß Salze von Alkenylbernsteinsäurehalbesteraminen der Formel Ila und/oder Ilb und Alkenylbernsteinsäureimide der Formel III in einer Konzentration bis zu 40 Gew.-%, bevorzugt bis zu 20 Gew.-%, im Gemisch mit den Salzen von Alkenylbernsteinsäure-alkoxyalkyl-halbamiden der Formel la und/oder Ib vorliegen.

3. Verfahren zur Herstellung von Salzen von Alkenylbernsteinsäurealkoxyalkyl-halbamiden der Formel la und/oder Ib gemäß Anspruch 1, gegebenenfalls in Mischungen mit Salzen von Alkenylbernsteinsäurehalbesteraminen der Formeln Ila und/oder Ilb und/oder Alkenylbernsteinsäureimiden der Formel III, dadurch gekennzeichnet, daß Alkenylbernsteinsäureanhydride mit Alkoxyalkylaminen der Formel IV 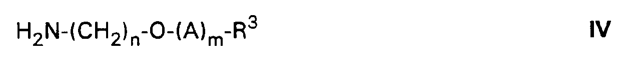 wobei
R³ = C₁-C₁₈-Alkyl, wobei R³ geradkettig oder verzweigt sein kann,
A = -CH₂-CH₂-O- oder -CH(R⁴)-CH₂-O- wobei
_{R}⁴ ₌ Wasserstoff oder Methyl,
n = eine Zahl von 1 bis 6 und
m = eine Zahl von 0 bis 8,
in einem molaren Verhältnis von 1 : 0,7 bis 2,5, vorzugsweise 1 : 0,8 bis 1,2, bei Temperaturen von 20 bis 50 °C, umgesetzt werden und diese Umsetzung in Gegenwart von 0,58 bis 0,60 Mol, bezogen auf Alkoxyalkylamin der Formel IV, von Basen erfolgt oder im Anschluß an die Umsetzung 0,58 bis 0,60 Mol, bezogen auf Alkoxyalkylamine der Formel IV, von Basen zugegeben wird, wobei als Basen Alkali- oder Erdalkalihydroxide sowie Alkanolamine der Formel HNR⁵R⁶R⁷ eingesetzt werden, wobei R⁵, R⁶ und R⁷ gleich oder verschieden sind und Wasserstoff, C₁-C₆-Alkyl oder Hydroxy-(C₁-C₆)-Alkyl bedeuten.

4. Verfahren nach Anspruch 3, dadurch gekennzeichnet, daß als eine der Basen NaOH eingesetzt wird.

5. Verfahren nach Anspruch 3, dadurch gekennzeichnet, daß als Alkoxyalkylamine der Formel IV 3-Methoxypropylamin und/oder 3-Butoxypropylamin eingesetzt werden.

6. Wasser- und/oder ölhaltige Formulierungen zur Bearbeitung von Metallen, insbesondere zur Verwendung als Kühlschmiermittel, dadurch gekennzeichnet, daß sie Salze von Alkenylbernsteinsäure-alkoxyalkylhalbamiden der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III, nach Anspruch 1 enthalten.

7. Formulierungen nach Anspruch 6, dadurch gekennzeichnet, daß die Verbindungen gemäß Anspruch 1 in einer Konzentration von 10 bis 80 Gew.-% enthalten sind.

8. Wasser- und/oder ölhaltige Metallbearbeitungsflüssigkeiten zur Bearbeitung von Metallen, insbesondere zur Verwendung als Kühlschmiermittel, dadurch gekennzeichnet, daß sie Salze von Alkenylbernsteinsäure-alkoxyalkyl- halbamiden der Formel la und/oder Ib, gegebenenfalls in Mischung mit Salzen von Alkenylbernsteinsäurehalbesteraminen der Formel Ila und/oder Ilb und/oder Alkenylbernsteinsäureimiden der Formel III, nach Anspruch 1 enthalten.

9. Wasser- und/oder ölhaltige Metallbearbeitungsflüssigkeiten nach Anspruch 8, dadurch gekennzeichnet, daß die Verbindungen nach Anspruch 1 in einer Konzentration von 0,1 bis 10 Gew.-%, bevorzugt 2 bis 10 Gew.-%, enthalten sind.

10. Formulierungen nach Anspruch 6, dadurch gekennzeichnet, daß als zusätzliche Hilfsstoffe Oxethylate der allgemeinen Formel V 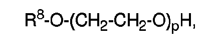 wobei R⁸ C_{1O}-C₂₂-Alkyl, C₁ₒ-C₂₂ Alkenyl oder Alkylphenyl mit insgesamt 10 bis 20 C-Atomen und p Zahlen von 2 bis 10 bedeuten und/oder Fettsäurealkanolamide der allgemeinen Formel VI 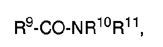 wobei R⁹ C_{1O}-C₂₂-Alkyl, C_{1O}-C₂₂-Alkenyl und R¹⁰ und R¹¹ gleich oder verschieden sind und Wasserstoff, Hydroxy-(C₁-C₆)-alkyl, insbesondere 2-Hydroxyethyl oder 2-Hydroxypropyl und/oder
Ethercarbonsäure der Formel VII 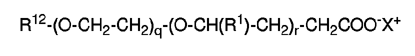 , wobei R¹² ein verzweigter oder unverzweigter Cₐ-C₂₂-Alkyl- oder Cₐ-C₂₂-Alkenylrest ist und q und r unabhängig voneinander Werte von 0 bis 10 besitzen, mit der Maßgabe, daß die Summe aus q und r mindestens 1 ist und X gleich Wasserstoff, ein Alkalimetallatom oder Erdalkalimetallatom bedeuten,
alkoxylierte primäre Amine der Formel VII 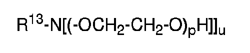 wobei
R13 C₁₂-C₂ₒ-Alkyl, oder Alkenyl vorzugsweise C₁₆-C₁₈-Alkyl,
p Zahlen von 2 bis 10, vorzugsweise 4 bis 6 und
u die Zahl 1 oder 2 bedeuten, mit der Maßgabe, daß falls u gleich 1 an der freien Bindung des Stickstoffatoms ein Wasserstoffatom sitzt,
und/oder
Fettsäurepolyglycolester, insbesondere von gesättigten oder ungesättigten Fettsäuren mit 10 bis 22 C-Atomen in der Alkylkette und 1 bis 10 Ethylenoxideinheiten, bevorzugt Ölsäure mit 4 bis 6 Ethylenoxideinheiten, enthalten sind.

11. Formulierungen nach Anspruch 10, dadurch gekennzeichnet, daß die Hilfsstoffe in einer Konzentration von 20 bis 40 Gew,-% enthalten sind.

12. Formulierungen nach Anspruch 6, dadurch gekennzeichnet, daß Entschäumer und/oder Konservierungsmittel enthalten sind.

13. Metalbearbeitungsflüssigkeiten nach Anspruch 8, dadurch gekennzeichnet, daß als zusätzliche Hilfsstoffe Oxethylate der allgemeinen Formel V 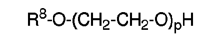 , wobei R⁸ C₁₀-C₂₂-Alkyl, C₁₀-C₂₂ Alkenyl oder Alkylphenyl mit insgesamt 10 bis 20 C-Atomen und p Zahlen von 2 bis 10 bedeuten und/oder Fettsäurealkanolamide der allgemeinen Formel VI 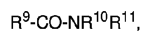 wobei R⁹ C₁₀-C₂₂-Alkyl, C₁ₒ-C₂₂-Alkenyl und R¹⁰ und R¹¹ gleich oder verschieden sind und Wasserstoff, Hydroxy-(C₁-C₆)-alkyl, insbesondere 2-Hydroxyethyl oder 2-Hydroxypropyl und/oder
Ethercarbonsäure der Formel VII 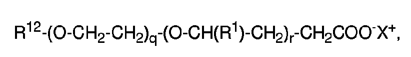 wobei R¹² ein verzweigter oder unverzweigter C₈-C₂₂-Alkyl- oder C₈-C₂₂-Alkenylrest ist und q und r unabhängig voneinander Werte von 0 bis 10 besitzen, mit der Maßgabe, daß die Summe aus q und r mindestens 1 ist und X gleich Wasserstoff, ein Alkalimetallatom oder Erdalkalimetallatom bedeuten,
alkoxylierte primäre Amine der Formel VIII 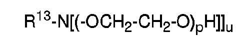 wobei
R¹³ C₁₂-C₂₀-Alkyl, oder Alkenyl vorzugsweise C₁₆-C₁₈-Alkyl,
p Zahlen von 2 bis 10, vorzugsweise 4 bis 6 und
u die Zahl 1 oder 2 bedeuten, mit der Maßgabe, daß falls u gleich 1 an der freien Bindung des Stickstoffatoms ein Wasserstoffatom sitzt,
und/oder
Fettsäurepolyglycolester, insbesondere von gesättigten oder ungesättigten Fettsäuren mit 10 bis 22 C-Atomen in der Alkylkette und 1 bis 10 Ethylenoxideinheiten, bevorzugt Ölsäure mit 4 bis 6 Ethylenoxideinheiten, enthalten sind.

14. Verwendung von Salzen von Alkenylbernsteinsäure-alkoxyalkyl-halbamiden der Formel la und/oder Ib, gegebenenfalls im Gemisch mit Salzen von Alkenylbernsteinsäurehalbesteraminen der Formel Ila und/oder Ilb und/oder Alkenylbernsteinsäureimiden der Formel III als Metallbearbeitungshilfsmittel, insbesondere als Korrosionsschutzmittel und Emulgator in wäßrigen und/oder ölhaltigen Formulierungen bzw. Metallbearbeitungshilfsmittel.

## Claims

1. A salt of an alkenylsuccinic acid alkoxyalkyl-half-amide of the formula la or Ib 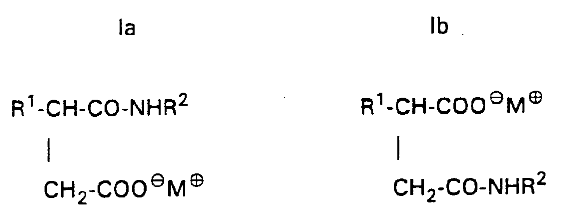 if appropriate as a mixture with a salt of an alkenylsuccinic acid half-ester amine of the formula Ila or Ilb 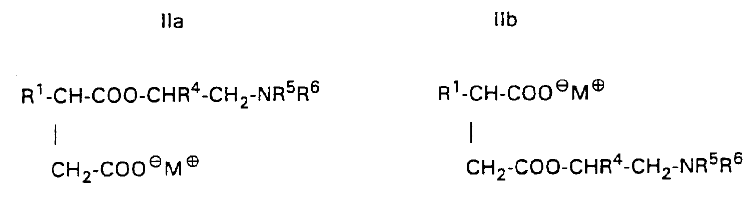 and/or an alkenylsuccinic acid imide of the formula III 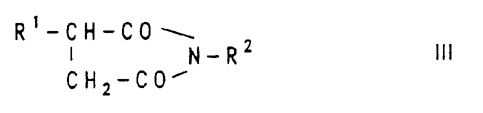 in which
R¹ is C₆-C₃₀-alkenyl or C₆-C₃₀-alkyl, preferably C₉-C₁₈-alkenyl or C₉-C₁₈-alkyl, where R¹ can be straight-chain or branched;
R² is -(CH₂)n-O-(A)m-R³, where
A is -CH₂-CH₂-O- or -CH(R⁴)-CH₂-0-, in which
R³ is C₁-C₁₈-alkyl, where R³ can be straight-chain or branched,
R⁴ is hydrogen or methyl,
n is a number from 1 to 6,
m is a number from 0 to 8 and
M⁺ is an alkali metal or alkaline earth metal cation or an ammonium ion of the formula HNR⁵R⁶R⁷, in which R⁵, R⁶ and R⁷ are identical or different and are hydrogen, C₁-C₆-alkyl or hydroxy-(C₁-C₆)-alkyl, in particular 2-hydroxyethyl or 2-hydroxypropyl.

2. A compound as claimed in claim 1, wherein a salt of an alkenylsuccinic acid half-ester amine of the formula Ila or Ilb and an alkenylsuccinic acid imide of the formula III are present in a concentration of up to 40 % by weight, preferably up to 20 % by weight, as a mixture with a salt of an alkenylsuccinic acid alkoxyalkyl-half-amide of the formula la or lb.

3. A process for the preparation of a salt of an alkenylsuccinic acid alkoxyalkyl-half-amide of the formula la or Ib as claimed in claim 1, if appropriate as a mixture with a salt of an alkenylsuccinic acid half-ester amine of the formula Ila or Ilb and/or an alkenylsuccinic acid imide of the formula III, which comprises reacting an alkenylsuccinic acid anhydride with an alkoxyalkylamine of the formula IV 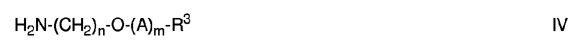 in which
R³ is C₁-C₁₈-alkyl, in which R³ can be straight-chain or branched,
A is -CH₂-CH₂-O- or -CH(R⁴)-CH₂-0-, in which
R⁴ is hydrogen or methyl,
n is a number from 1 to 6 and
m is a number from 0 to 8,
in a molar ratio of 1 : 0.7 to 2.5, preferably 1 : 0.8 to 1.2, at temperatures of 20 to 50°C, and carrying out this reaction in the presence of from 0.58 to 0.60 mol, based on alkoxyalkylamine of the formula IV, of bases or adding from 0.58 to 0.60 mol, based on alkoxyalkylamine of the formula IV, of bases after the reaction, the bases employed being alkali metal or alkaline earth metal hydroxides and alkanolamines of the formula HNR⁵R⁶R⁷, in which R⁵, R⁶ and R⁷ are identical or different and are hydrogen, C₁-C₆-alkyl or hydroxy-(C₁-C₆)-alkyl.

4. The process as claimed in claim 3, wherein NaOH is employed as one of the bases.

5. The process as claimed in claim 3, wherein 3-methoxypropylamine and/or 3-butoxypropylamine are employed as the alkoxyalkylamine of the formula IV.

6. An aqueous or oil-containing formulation for working metals, in particular for use as a cooling lubricant, which comprises a salt of an alkenylsuccinic acid alkoxyalkyl-half-amide of the formula la or Ib, if appropriate as a mixture with a salt of an alkenylsuccinic acid half-ester amine of the formula Ila or Ilb and/or an alkenylsuccinic acid imide of the formula III, as claimed in claim 1.

7. A formulation as claimed in claim 6, which comprises a compound as claimed in claim 1 in a concentration of 10 to 80 % by weight.

8. An aqueous or oil-containing metalworking fluid for working metals, in particular for use as a cooling lubricant, which comprises a salt of an alkenylsuccinic acid alkoxyalkyl-half-amide of the formula la or Ib, if appropriate as a mixture with a salt of an alkenylsuccinic acid half-ester amine of the formula Ila or Ilb and/or an alkenylsuccinic acid imide of the formula III, as claimed in claim 1.

9. An aqueous or oil-containing metalworking fluid as claimed in claim 8, which comprises a compound as claimed in claim 1 in a concentration of 0.1 to 10 % by weight, preferably 2 to 10 % by weight.

10. A formulation as claimed in claim 6, which comprises as additional auxiliaries an oxyethylate of the formula V 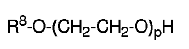 in which R⁸ is C₁₀-C₂₂-alkyl, C₁₀-C₂₂ alkenyl or alkylphenyl having a total of 10 to 20 carbon atoms and p is a number from 2 to 10, and/or a fatty acid alkanolamide of the formula VI 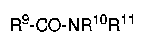 in which R⁹ is C₁₀-C₂₂-alkyl or C₁₀-C₂₂-alkenyl and R¹⁰ and R¹¹ are identical or different and are hydrogen or hydroxyl-(C₁-C₆)-alkyl, in particular 2-hydroxyethyl or 2-hydroxypropyl, and/or
an ether-carboxylic acid of the formula VII 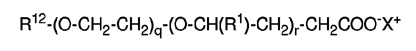 in which R¹² is a branched or unbranched C₈-C₂₂-alkyl or C₈-C₂₂-alkenyl radical and q and r independently of one another have values from 0 to 10, with the proviso that the sum of q and r is at least 1, and X is hydrogen, an alkali metal atom or an alkaline earth metal atom,
an alkoxylated primary amine of the formula VIII 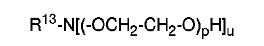 in which
R¹³ is C₁₂-C₂ₒ-alkyl, or alkenyl, preferably C₁₆-C₁₈-alkyl,
p is a number from 2 to 10, preferably 4 to 6, and
u is the number 1 or 2, with the proviso that if u is 1, a hydrogen atom is located on the free bond of the nitrogen atom,
and/or
a fatty acid polyglycol ester, in particular of a saturated or unsaturated fatty acid having 10 to 22 carbon atoms in the alkyl chain and 1 to 10 ethylene oxide units, preferably oleic acid having 4 to 6 ethylene oxide units.

11. A formulation as claimed in claim 10, which comprises the auxiliaries in a concentration of 20 to 40 % by weight.

12. A formulation as claimed in claim 6, which comprises a foam suppressant and/or preservative.

13. A metalworking fluid as claimed in claim 8, which comprises as additional auxiliaries an oxyethylate of the formula V 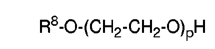 in which R⁸ is C₁₀-C₂₂-alkyl, C₁₀-C₂₂ alkenyl or alkylphenyl having a total of 10 to 20 carbon atoms and p is a number from 2 to 10, and/or a fatty acid alkanolamide of the formula VI 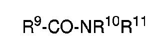 in which _{R}⁹ is C₁₀-C₂₂-alkyl or C₁₀-C₂₂-alkenyl and R¹⁰ and R¹¹ are identical or different and are hydrogen or hydroxyl-(C₁-C₆)-alkyl, in particular 2-hydroxyethyl or 2-hydroxypropyl, and/or
an ether-carboxylic acid of the formula VII 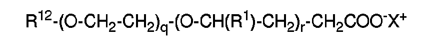 in which R¹² is a branched or unbranched, C₈-C₂₂-alkyl or C₈-C₂₂-alkenyl radical and q and r independently of one another have values from 0 to 10, with the proviso that the sum of q and r is at least 1, and X is hydrogen, an alkali metal atom or an alkaline earth metal atom,
an alkoxylated primary amine of the formula VIII 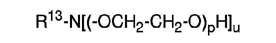 in which
R¹³ is C₁₂-C₂ₒ-alkyl, or alkenyl, preferably C₁₆-C₁₈-alkyl,
p is a number from 2 to 10, preferably 4 to 6, and
u is the number 1 or 2, with the proviso that if u is 1, a hydrogen atom is located on the free bond of the nitrogen atom,
and/or
a fatty acid polyglycol ester, in particular of a saturated or unsaturated fatty acid having 10 to 22 carbon atoms in the alkyl chain and 1 to 10 ethylene oxide units, preferably oleic acid having 4 to 6 ethylene oxide units.

14. The use of a salt of an alkenylsuccinic acid alkoxyalkyl-half-amide of the formula la or Ib, if appropriate as a mixture with a salt of an alkenylsuccinic acid half-ester amine of the formula Ila or Ilb and/or an alkenylsuccinic acid imide of the formula III, as a metalworking auxiliary, in particular as an anticorrosion agent and emulsifier in an aqueous or oil-containing formulation or metalworking auxiliary

## Revendications

1. Sels d'alcoxyalkyl-semi-amides d'acides alcénylsucciniques de formule la et/ou Ib : 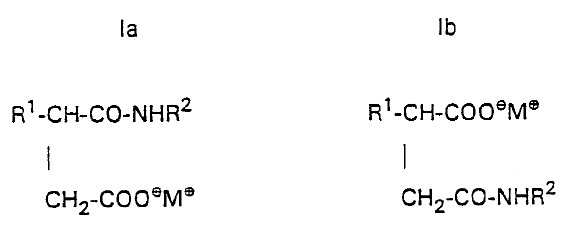 éventuellement en mélange avec des sels de semi-ester-amines d'acides alcénylsucciniques de formule Ila et/ou Ilb: 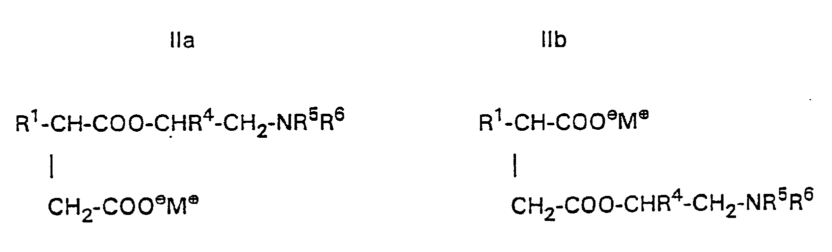 et/ou d'alcénylsuccinimides de formule III : 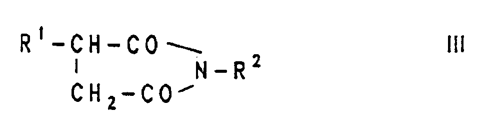 où :
R¹ est un groupe alcényle en C₆-C_{30'} ou alkyle en C₆-C₃ₒ, de préférence alcényle en C₉-C_{18'} ou alkyle en Cg-C₁₈, R¹ pouvant avoir une chaîne droite ou ramifiée ;
R² est -(CH₂)ₙ-O-(A)ₘ-R³, où
A est - CH₂-CH₂-O- ou -CH(R⁴)-CH₂-O-, où
R³ est un groupe alkyle en C₁-C_{18'} R³ pouvant avoir une chaîne droite ou ramifiée,
R⁴ est un atome d'hydrogène ou le groupe méthyle,
n est un nombre de 1 à 6,
m est un nombre de 0 à 8, et
M⁺ est un cation d'un métal alcalin ou alcalino-terreux, ainsi qu'un ion ammonium de formule HNR⁵R⁶R⁷, où les radicaux R⁵, R⁶ et R⁷ sont identiques ou différents et représentent chacun un atome d'hydrogène, un groupe alkyle en C₁-C₆ ou hydroxy-(alkyle en C₁-C6), en particulier 2-hydroxyéthyle et 2-hydroxypropyle.

2. Composés selon la revendication 1, caractérisés en ce que les sels des semi-esteramines des acides alcénylsucciniques de formule Ila et/ou Ilb et des alcénylsuccinimides de formule III sont présents à une concentration allant jusqu'à 40 % en poids, de préférence jusqu'à 20 % en poids, en mélange avec les sels des alcoxy-alkyl-semi-amides d'acides alcénylsucciniques de formule la et/ou Ib.

3. Procédé de préparation de sels d'alcoxyalkyl-semi-amides d'acides alcénylsucciniques de formule la et/ou Ib selon la revendication 1, éventuellement en mélange avec des sels de semi-esteramines d'acides alcénylsucciniques de formule Ila et/ou Ilb et/ou d'alcénylsuccinimides de formule III, caractérisé en ce qu'on fait réagir des anhydrides alcénylsucciniques avec des alcoxyalkylamines de formule IV : 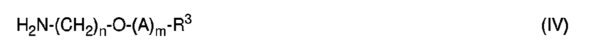 dans laquelle :
R³ est un groupe alkyle en C₁-C_{18'} R³ pouvant avoir une chaîne droite ou ramifiée,
A est -CH₂-CH₂-O ou -CH(R⁴)-CH₂-0-, où
R⁴ est un atome d'hydrogène ou un groupe méthyle,
n est un nombre de 1 à 6, et
m est un nombre de 0 à 8,
selon un rapport en moles de 1:0,7 à 2,5 et de préférence de 1:0,8 à 1,2, à des températures de 20 à 50°C, cette réaction est réalisée en présence de 0,58 à 0,60 mole de bases par rapport à l'alcoxyalkylamine de formule IV, ou encore, après la réaction, on ajoute de 0,58 à 0,60 mole de bases, par rapport aux alcoxyalkylamines de formule IV, auquel cas on utilise en tant que bases des hydroxydes de métaux alcalins ou alcalino-terreux ainsi que des alcanolamines de formule HNR⁵R⁶R⁷, où R⁵, R⁶ et R⁷ sont identiques ou différents et représentent chacun un atome d'hydrogène, un groupe alkyle en Ci-C₆ ou hydroxyalkyle en C₁-C₆.

4. Procédé selon la revendication 3, caractérisé en ce qu'on utilise du NaOH en tant que l'une des bases.

5. Procédé selon la revendication 3, caractérisé en ce qu'on utilise en tant qu'alcoxyalkylamines de formule IV la 3-méthoxypropylamine et/ou la 3-butoxypropylamine.

6. Formulations contenant de l'eau et/ou une huile pour l'usinage des métaux, en particulier pour utilisation en tant que lubrifiants réfrigérants, caractérisées en ce qu'elles contiennent des sels d'alcoxyalkyl-semi-amides d'acides alcénylsucciniques de formule la et/ou Ib, éventuellement en mélange avec des sels de semi-esteramines d'acides alcénylsucciniques de formule Ila et/ou Ilb et/ou d'alcénylsuccinimides de formule III, selon la revendication 1.

7. Formulationsselon la revendication 6, caractérisée en ce que les composés selon la revendication 1 sont présents à une concentration de 10 à 80 % en poids.

8. Liquides contenant de l'eau et/ou une huile pour l'usinage des métaux, pour l'usinage de métaux, en particulier pour utilisation en tant que lubrifiants-réfrigérants, caractérisés en ce qu'ils contiennent des sels d'alcoxyalkyl- semi-amides d'acides alcénylsucciniques de formule la et/ou Ib, éventuellement en mélange avec des sels de semi-esteramines d'acides alcénylsucciniques de formule Ila et/ou Ilb et/ou d'alcénylsuccinimides de formule III, selon la revendication 1.

9. Liquides pour l'usinage des métaux, contenant de l'eau et/ou une huile, selon la revendication 8, caractérisés en ce qu'ils contiennent les composés selon la revendication 1 à une concentration de 0,1 à 10 % en poids et de préférence de 2 à 10 % en poids.

10. Formulations selon la revendication 6, caractérisées en ce qu'elles contiennent en tant qu'adjuvants additionnels
des produits d'éthoxylation de formule générale V : 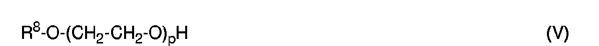 dans laquelle R⁸ est un groupe alkyle en C₁₀-C₂₂, alcényle en C₁₀-C₂₂ ou alkylphényle ayant en tout de 10 à 20 atomes de carbone, et p est un nombre de 2 à 10, et/ou
des alcanolamides d'acides gras de formule générale VI : 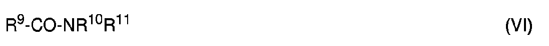 dans laquelle R⁹ est un groupe alkyle en C₁₀-C_{22'} alcényle en C₁₀-C₂₂, et R¹⁰ et R¹¹ sont identiques ou différents et représentent chacun un atome d'hydrogène, un groupe hydroxyalkyle en Ci-Ce, en particulier 2-hydroxyéthyle ou 2-hydroxypropyle, et/ou
un acide éther-carboxylique de formule VII : 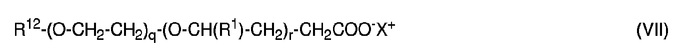 dans laquelle R¹² est un groupe alkyle en C₈-C₂₂ ou alcényle en C₈-C₂₂ à chaîne droite ou ramifiée, et q et r représentent chacun, indépendamment l'un de l'autre, des nombres de 0 à 10, à la condition que la somme de q et de r soit d'au moins 1 et que X soit un atome d'hydrogène, un atome d'un métal alcalin ou d'un métal alcalino-terreux,
des amines primaires alcoxydées de formule VIII : 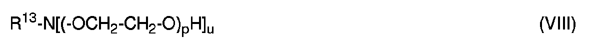 dans laquelle :
R¹³ est un groupe alkyle en C₁-C₂₀, ou alcényle, de préférence alkyle en C₁₆-Cₗ₈,
p est un nombre de 2 à 10, de préférence de 4 à 6, et
u vaut 1 ou 2, à la condition que, si u vaut 1, un atome d'hydrogène se trouve sur la liaison libre de l'atome d'azote, et/ou
des esters d'acides gras du polyglycol, en particulier d'acides gras saturés ou insaturés ayant de 10 à 22 atomes de carbone dans la chaîne alkyle et de 1 à 10 motifs oxyde d'éthylène, de préférence l'acide oléique avec de 4 à 6 motifs oxyde d'éthylène.

11. Formulations selon la revendication 10, caractérisées en ce que les adjuvants sont présents à une concentration de 20 à 40 % en poids.

12. Formulations selon la revendication 6, caractérisées en ce qu'elles contiennent des antimoussants et/ou des conservateurs.

13. Liquides pour l'usinage des métaux selon la revendication 8, caractérisés en ce qu'ils contiennent en tant qu'adjuvants additionnels
des produits d'éthoxylation de formule générale V : 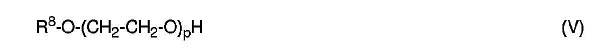 dans laquelle R⁸ est un groupe alkyle en C₁₀-C₂₂, alcényle en C₁₀-C₂₂ ou alkylphényle ayant en tout de 10 à 20 atomes de carbone, et p est un nombre de 2 à 10, et/ou
des alcanolamides d'acides gras de formule générale VI : 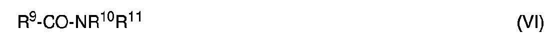 dans laquelle R⁹ est un groupe alkyde en C₁₀-C₂₂, alcényle en C₁₀-C₂₂, et R¹⁰ et R¹¹ sont identiques ou différents et représentent chacun un atome d'hydrogène, un groupe hydroxyalkyle en C₁-C₆, en particulier 2-hydroxyéthyle ou 2-hydroxypropyle, et/ou
un acide éther-carboxylique de formule VII : 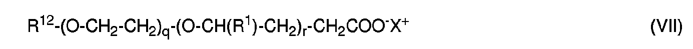 dans laquelle R¹² est un groupe alkyle en Cₐ-C₂₂ ou alcényle en C₈-C₂₂ à chaîne droite ou ramifiée, et q et r représentent chacun indépendamment l'un de l'autre, des nombres de 0 à 10, à la condition que la somme de q et de r soit d'au moins 1 et que X soit un atome d'hydrogène, un atome d'un métal alcalin ou d'un métal alcalino-terreux,
des amines primaires alcoxylées de formule VIII : 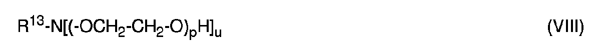 dans laquelle :
R¹³ est un groupe alkyle en C₁-C₂₀, ou alcényle, de préférence alkyle en C₁₆-C₁₈,
p est un nombre de 2 à 10, de préférence de 4 à 6, et
u vaut 1 ou 2, à la condition que, si u vaut 1, un atome d'hydrogène se trouve sur la liaison libre de l'atome d'azote, et/ou
des esters d'acides gras du polyglycol, en particulier d'acides gras saturés ou insaturés ayant de 10 à 22 atomes de carbone dans la chaîne alkyle et de 1 à 10 motifs oxyde d'éthylène, de préférence d'acide oléique avec de 4 à 6 motifs oxyde d'éthylène.

14. Utilisation de sels d'alcoxyalkyl-semi-amides d'acides alcénylsucciniques de formule la et/ou Ib, éventuellement en mélange avec des sels de semi-esteramines d'acides alcénylsucciniques de formule Ila et/ou Ilb et/ou d'alcénylsuccinimides de formule III en tant qu'adjuvants pour l'usinage des métaux, en particulier en tant qu'agents anticorrosion et émulsifiants dans des formulations ou adjuvants pour l'usinage des métaux, aqueux et/ou contenant une huile.
